(19) Europäisches Patentamt
European Patent Office
Office européen des brevets

(11) Veröffentlichungsnummer: **0 344 500**
**A1**

(12) **EUROPÄISCHE PATENTANMELDUNG**

(21) Anmeldenummer: **89108651.4**

(22) Anmeldetag: **13.05.89**

(51) Int. Cl.⁴: **C07D 401/06 , C07D 403/06 , C07D 405/06 , C07D 409/06 , C07D 413/06 , C07D 417/06 , A01N 43/54**

(30) Priorität: **28.05.88 DE 3818163**

(43) Veröffentlichungstag der Anmeldung:
**06.12.89 Patentblatt 89/49**

(84) Benannte Vertragsstaaten:
**BE CH DE FR GB IT LI NL**

(71) Anmelder: **BAYER AG**
**D-5090 Leverkusen 1 Bayerwerk(DE)**

(72) Erfinder: **Diehr, Hans-Joachim, Dr.**
**Höhe 35**
**D-5600 Wuppertal 1(DE)**
Erfinder: **Becker, Benedikt, Dr.**
**Metzkausener Strasse 14**
**D-4020 Mettmann(DE)**

(54) **3-Substituierte 1-Nitro-2-imino-1,3-diazacycloalkane.**

(57) Die vorliegende Erfindung betrifft neue 3-substituierte 1-Nitro-2-imino-1,3-diazacycloalkane der Formel (I)

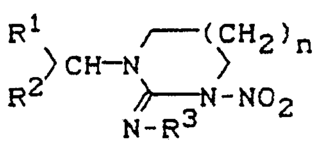

(I)

in welcher
n für die Zahlen 0 oder 1 steht,
$R^1$ für eine gegebenenfalls substituierte, fünf- oder sechsgliedrige heterocyclische Gruppierung steht, welche 1, 2, 3 oder 4 Stickstoffatome und/oder ein oder zwei Sauerstoff- oder Schwefelatome als Heteroatom-Ringglieder enthält - wobei die Zahl der Heteroatome 1, 2, 3 oder 4 beträgt,
$R^2$ für Wasserstoff oder Alkyl steht und
$R^3$ für Wasserstoff oder Nitro steht.
Die Erfindung betrifft weiterhin Verfahren zur Herstellung der Verbindungen (I) und ihre Verwendung als Schädlingsbekämpfungsmittel, insbesondere als Insektizide.

EP 0 344 500 A1

## 3-Substituierte 1-Nitro-2-imino-1,3-diazacycloalkane

Die vorliegende Erfindung betrifft neue 3-substituierte 1-Nitro-2-imino-1,3-diazacycloalkane, Verfahren zu ihrer Herstellung und ihre Verwendung als Schädlingsbekämpfungsmittel, insbesondere als Insektizide.

Es ist bereits bekannt geworden, daß bestimmte organische Nitroverbindungen, wie z. B. 2-Nitromethylen-2H-tetrahydro-1,3-thiazin, insektizide Eigenschaften aufweisen (vgl. US-PS 3 993 648).

Es wurden nun die neuen 3-substituierten 1-Nitro-2-imino-1,3-diaza-cycloalkane der allgemeinen Formel (I)

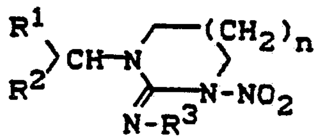

(I)

in welcher
n für die Zahlen 0 oder 1 steht,
$R^1$ für eine fünf- oder sechsgliedrige heterocyclische Gruppierung steht, welche 1, 2, 3 oder 4 Stickstoffatome und/oder ein oder zwei Sauerstoff- oder Schwefelatome als Heteroatom-Ringglieder enthält - wobei die Zahl der Heteroatome 1, 2, 3 oder 4 beträgt - und welche gegebenenfalls durch Halogen, Cyano, Nitro, Alkyl, Halogenalkyl, Alkenyl, Halogenalkenyl, Alkinyl, Alkoxy, Halogenalkoxy, Alkenyloxy, Halogenalkenyloxy, Alkinyloxy, Alkylthio, Halogenalkythio, Alkenylthio, Halogenalkenylthio, Alkinylthio, Alkylsulfinyl, Halogenalkylsulfinyl, Alkylsulfonyl, Halogenalkylsulfonyl, Amino, Alkylamino, Dialkylamino, Aryl, Aryloxy, Arylthio, Arylamino, Aralkyl, Formylamino, Alkylcarbonylamino, Formyl, Carbamoyl, Alkylcarbonyl und/oder Alkoxycarbonyl substituiert ist,
$R^2$ für Wasserstoff oder Alkyl steht und
$R^3$ für Wasserstoff oder Nitro steht,
gefunden.

Man erhält die neuen 3-substituierten 1-Nitro-2-imino-1,3-diaza-cycloalkane der allgemeinen Formel (I), wenn man

(a) 1-substituierte 2-Imino-1,3-diaza-cycloalkane der allgemeinen Formel (II),

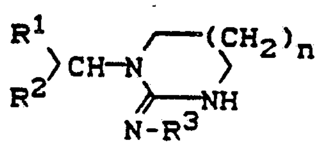

(II)

in welcher
n, $R^1$, $R^2$ und $R^3$ die oben angegebenen Bedeutungen haben,
-oder deren Hydrochloride-
mit einem Nitrierungsmittel, gegebenenfalls in Gegenwart eines Katalysators und gegebenenfalls in Gegenwart eines Verdünnungsmittels umsetzt, oder wenn man
-für den Fall, daß $R^3$ für Wasserstoff steht-

(b) 1-substituierte 2-Nitroimino-1,3-diazacycloalkane der allgemeinen Formel (IIa),

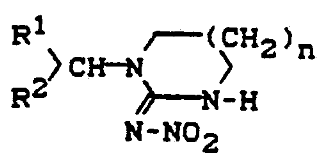

(IIa)

in welcher
n, $R^1$ und $R^2$ die oben angegebenen Bedeutungen haben,
gegebenenfalls in Gegenwart eines Katalysators und gegebenenfalls in Gegenwart eines Verdünnungsmittels, isomerisiert.

Die neuen 3-substituierten 1-Nitro-2-imino-1,3-diazacycloalkane der allgemeinen Formel (I) zeichnen sich durch starke insektizide Wirksamkeit aus.

Überraschenderweise zeigen die erfindungsgemäßen Verbindungen der Formel (I) erheblich stärkere insektizide Wirkung als nach Struktur und Wirkprofil vergleichbare organische Nitroverbindungen, wie z.B. 2-Nitromethylen-2H-tetrahydro-1,3-thiazin.

Die Erfindung betrifft vorzugsweise Verbindungen der Formel (I), in welcher

n für die Zahlen 0 oder 1 steht,

$R^1$ für eine fünf- bzw. sechsgliedrige heterocyclische Gruppierung aus der Reihe Furyl, Thienyl, Pyrrolyl, Pyrazolyl, Imidazolyl, 1,2,3- oder 1,2,4-Triazolyl, Oxazolyl, Isoxazolyl, 1,2,4- oder 1,3,4-Oxadiazolyl, Thiazolyl, Isothiazolyl, 1,2,3- 1,2,4,-1,2,5- oder 1,3,4-Thiadiazolyl, Pyridyl, Pyridazinyl, Pyrimidinyl and Pyrazinyl steht, welche gege benenfalls durch Fluor, Chlor, Brom, Iod, Cyano, Nitro, $C_1$-$C_4$-Alkyl (welches gegebenenfalls durch Fluor und/oder Chlor substituiert ist), $C_2$-$C_4$-Alkenyl (welches gegebenenfalls durch Fluor und/oder Chlor substituiert ist), $C_2$-$C_4$-Alkinyl, $C_1$-$C_4$-Alkoxy (welches gegebenenfalls durch Fluor und/oder Chlor substituiert ist), $C_3$-$C_4$-Alkenyloxy (welches gegebenenfalls durch Fluor und/oder Chlor substituiert ist), $C_3$-$C_4$-Alkinyloxy, $C_1$-$C_4$-Alkylthio (welches gegebenenfalls durch Fluor und/oder Chlor substituiert ist), $C_3$-$C_4$-Alkenylthio (welches gegebenenfalls durch Fluor und/oder Chlor substituiert ist), $C_3$-$C_4$-Alkinylthio, $C_1$-$C_4$-Alkylsulfinyl (welches gegebenenfalls durch Fluor und/oder Chlor substituiert ist), $C_1$-$C_4$-Alkylsulfonyl (welches gegebenenfalls durch Fluor und/oder Chlor substituiert ist), Amino, $C_1$-$C_4$-Alkylamino, Di-($C_1$-$C_4$-alkyl)-amino, Phenyl, Phenoxy, Phenylthio, Phenylamino, Benzyl, Formylamino, $C_1$-$C_4$-Alkyl-carbonylamino, Formyl, Carbamoyl, $C_1$-$C_4$-Alkyl-carbonyl und/oder $C_1$-$C_4$-Alkoxy-carbonyl substituiert ist,

$R^2$ für Wasserstoff oder $C_1$-$C_3$-Alkyl steht und

$R^3$ für Wasserstoff oder Nitro steht.

Die Erfindung betrifft insbesondere Verbindungen der Formel (I), in welcher

n für die Zahlen 0 oder 1 steht,

$R^1$ für eine fünf- bzw. sechsgliedrige heterocyclische Gruppierung aus der Reihe Pyrazolyl, 1,2,4-Triazolyl, Oxazolyl, Isoxazolyl, Thiazolyl, Isothiazolyl, 1,2,5-Thiadiazolyl, Pyridyl, Pyrazinyl und Pyrimidinyl steht, welche gegebenenfalls durch Fluor, Chlor, Brom, Cyano, Nitro, $C_1$-$C_2$-Alkyl (welches gegebenenfalls durch Fluor und/oder Chlor substituiert ist), $C_1$-$C_2$-Alkoxy (welches gegebenenfalls durch Fluor und/oder Chlor substituiert ist), $C_1$-$C_2$-Alkylthio (welches gegebenenfalls durch Fluor und/oder Chlor substituiert ist) oder $C_1$-$C_2$-Alkylsulfonyl (welches gegebenenfalls durch Fluor und/oder Chlor substituiert ist) substituiert ist,

$R^2$ für Wasserstoff steht,

$R^3$ für Wasserstoff oder Nitro steht.

Verwendet man als Ausgangsstoffe beispielsweise 1-(2-Chlor-thiazol-5-yl-methyl)-2-nitroiminoimidazolidin und Salpetersäure, so kann der Reaktionsablauf beim erfindungsgemäßen Verfahren (a) durch das folgende Formelschema skiziert werden:

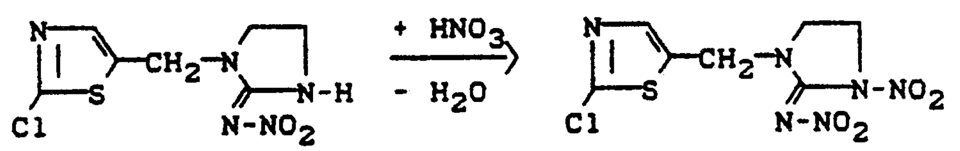


Verwendet man als Ausgangsstoff ebenfalls 1-(2-Chlorthiazol-5-yl-methyl)-2-nitroiminoimidazolidin, so kann der Reaktionsablauf beim erfindungsgemäßen Verfahren (b) durch das folgende Formelschema skizziert werden:

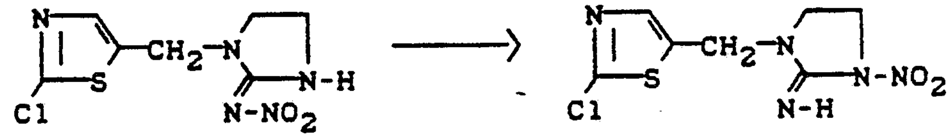

Die beim erfindungsgemäßen Verfahren (a) zur Herstellung von Verbindungen der Formel (I) als Ausgangsstoffe zu verwendenden 1-substituierten 2-Imino-1,3-diaza-cycloalkane sind durch die Formel (II) allgemein definiert.

In Formel (II) haben n, $R^1$, $R^2$ und $R^3$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für n, $R^1$, $R^2$ und $R^3$ angegeben wurden.

Beispiele für die Ausgangsstoffe der Formel (II) sind in der nachstehenden Tabelle 1 aufgeführt.

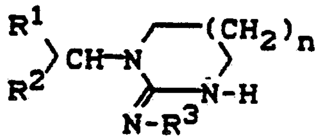

(II)

Tabelle 1: Beispiele für Ausgangsstoffe der Formel (II)

| n | $R^1$ | $R^2$ | $R^3$ |
|---|---|---|---|
| 0 | 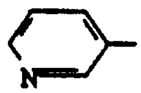 | H | H |
| 0 | 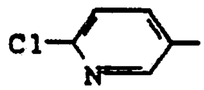 | H | H |
| 0 | 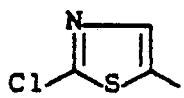 | H | H |
| 1 | 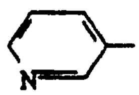 | H | H |
| 1 | 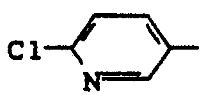 | H | H |
| 1 | 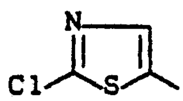 | H | H |
| 0 | 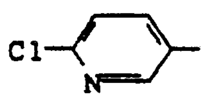 | H | $NO_2$ |
| 0 | 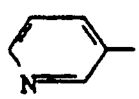 | H | $NO_2$ |
| 0 | 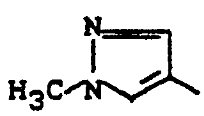 | H | H |
| 0 | 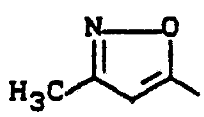 | H | $NO_2$ |

<u>Tabelle 1</u> - Fortsetzung

| n | $R^1$ | $R^2$ | $R^3$ |
|---|---|---|---|
| 0 | 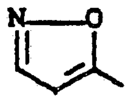 | H | H |
| 0 | 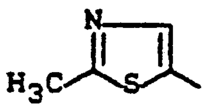 | H | H |
| 0 | 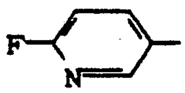 | H | H |
| 1 | 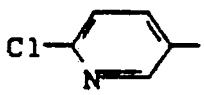 | H | $NO_2$ |
| 0 | 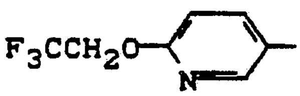 | H | H |
| 0 | 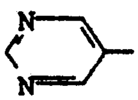 | H | H |
| 0 | 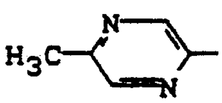 | H | H |
| 0 | 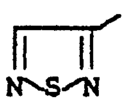 | H | H |
| 0 | 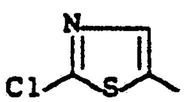 | H | $NO_2$ |
| 0 | 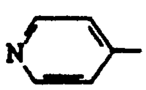 | H | $NO_2$ |

**Tabelle 1 - Fortsetzung**

| n | $R^1$ | $R^2$ | $R^3$ |
|---|---|---|---|
| 1 | 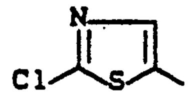 | H | $NO_2$ |
| 0 | 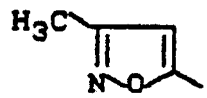 | H | H |
| 1 | 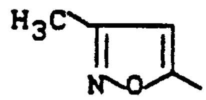 | H | $NO_2$ |
| 0 | 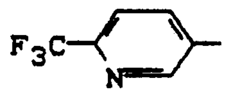 | H | H |
| 0 | 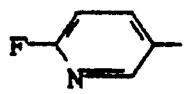 | H | $NO_2$ |
| 0 | 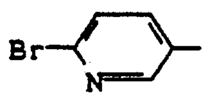 | H | H |

Die beim erfindungsgemäßen Verfahren (b) zur Herstellung von Verbindungen der Formel (I) als Ausgangsstoffe zu verwendenden 1-substituierten 2-Nitroimino-1,3-diazacycloalkane sind durch die Formel (IIa) allgemein definiert.

In Formel (IIa) haben n, $R^1$ und $R^2$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für n, $R^1$ und $R^2$ angegeben wurden.

Beispiele für die Ausgangsstoffe der Formel (IIa) sind Tabelle 1 ($R^3$:$NO_2$) zu entnehmen.

Die Ausgangsstoffe der Formeln (II) und (IIa) sind bekannt und/oder können nach an sich bekannten Verfahren hergestellt werden (vgl. EP-A 192 060).

Das erfindungsgemäße Verfahren (a) wird unter Einsatz eines Nitrierungsmittels durchgeführt. Es können die üblichen zur Nitrierung von organischen Verbindungen geeigneten Stoffe verwendet werden. Vorzugsweise wird Salpetersäure als Nitrierungsmittel eingesetzt.

Die erfindungsgemäßen Verfahren (a) und (b) werden vorzugsweise in Gegenwart eines Katalysators durchgeführt. Als solche kommen Säuren, wie Schwefelsäure, Methansulfonsäure oder Essigsäure, Säureanhydride, wie Acetanhydrid und/oder Salze von Übergangsmetallen, wie Kupfersulfat oder -nitrat, in Betracht. Vorzugsweise wird Schwefelsäure als Katalysator verwendet.

Die erfindungsgemäßen Verfahren (a) und (b) werden vorzugsweise in Gegenwart eines Verdünnungsmittels durchgeführt. Als solche kommen Säureanhydride, wie z.B. Acetanhydrid, Säuren, wie Schwefelsäure, Methansulfonsäure oder Essigsäure, inerte organische Solventien, wie Methylenchlorid, sowie Wasser in Betracht.

Die Reaktionstemperaturen können bei den erfindungsgemäßen Verfahren (a) und (b) in einem größeren Bereich variiert werden. Im allgemeinen arbeitet man bei Temperaturen zwischen - 20 °C und + 80 °C, vorzugsweise bei Temperaturen zwischen 0 °C und 50 °C.

Die erfindungsgemäßen Verfahren (a) und (b) werden im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) setzt man je Mol Ausgangsverbindung der Formel (II) im allgemeinen zwischen 1 und 5 Mol, vorzugsweise zwischen 1 und 2 Mol, eines Nitrierungsmittels ein.

Die Reaktionskomponenten werden im allgemeinen unter leichtem Kühlen vermischt und bei Raumtemperatur oder leicht erhöhter Temperatur bis zum Ende der Umsetzung gerührt. Die Aufarbeitung kann nach üblichen Methoden durchgeführt werden.

Das erfindungsgemäße Verfahren (b) kann -vom Nitrierungsmittel abgesehen- auf gleiche Weise wie Verfahren (a) durchgeführt werden.

Die Wirkstoffe eignen sich zur Bekämpfung von tierischen Schädlingen, vorzugsweise Arthropoden insbesondere Insekten die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Die erfindungsgemäßen Verbindungen der Formel (I) zeichnen sich durch hervorragende insektizide Wirksamkeit aus. Sie zeigen insbesondere beim Einsatz gegen pflanzenschädigende Insekten, insbesondere gegen Blattinsekten, daneben aber auch (wurzelsystemisch) gegen Bodeninsekten, starke Wirkung.

Einige der erfindungsgemäßen Verbindungen zeigen auch fungizide Wirkung, beispielsweise gegen falsche Mehltaupilze und gegen Reisbrennen (Pyricularia oryzae).

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formu lierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarb stoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Die Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Herstellungsbeispiele

Beispiel 1

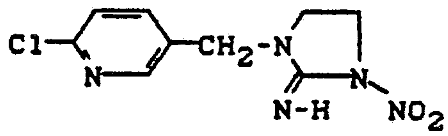

(Verfahren a)

24,7 g (0,1 Mol) 1-(2-Chlor-pyridin-5-yl-methyl)-2-imino-imidazolidin-Hydrochlorid werden bei 10 °C bis 20 °C in 80 ml 96 %iger Schwefelsäure suspendiert. Der dabei freigesetzte Chlorwasserstoff wird im Wasserstrahlvakuum abgezogen und die dabei entstandene klare Lösung wird bei 10 °C bis 20 °C mit 10,3 g (0,16 Mol) 98 %iger Salpetersäure versetzt. Das Reaktionsgemisch wird 12 Stunden bei 20 °C gerührt, mit Eis vermischt, mit Natronlauge neutralisiert und mit Methylenchlorid extrahiert. Die organische Phase wird mit Wasser gewaschen, mit Natriumsulfat getrocknet und filtriert. Vom Filtrat wird das Lösungsmittel im Wasserstrahlvakuum sorgfältig abdestilliert.

Man erhält 21,3 g (83,5 % der Theorie) 1-(2-Chlorpyridin-5-yl-methyl)-2-imino-3-nitro-imidazolidin als öligen Rückstand, welcher bei Anreiben kristallisiert. Schmelzpunkt: 81 °C.

Beispiel 2

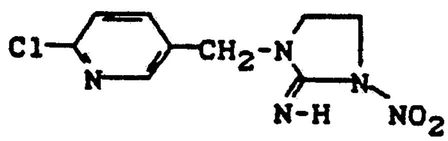

(Verfahren b)

63,9 g (0,25 Mol) 1-(2-Chlor-pyridin-5-yl-methyl)-2-nitroimino-imidazolidin werden in 250 ml 96 %iger Schwefelsäure bei 20 °C bis 25 °C -Außenkühlung mit Eiswasser erforderlich- suspendiert. Das Reaktionsgemisch wird 12 Stunden bei 20 °C gerührt und -wie bei Beispiel 1 beschrieben- aufgearbeitet.

Man erhält 53,5 g (84 % der Theorie) 1-(2-Chlor-pyridin-5-yl-methyl)-2-imino-3-nitro-imidazolidin vom Schmelzpunkt 81 °C.

Beispiel 3

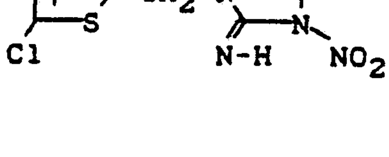

(Verfahren a)

(a) Analog Beispiel 1 wurden aus 12,7 g (0,05 Mol) 1-(2-Chlor-thiazol-5-yl-methyl)-2-iminoimidazolidin-Hydrochlorid, 40 ml 96 %iger Schwefelsäure und 5,1 g 98 %iger Salpetersäure 3,2 g (27 % der Theorie) 1-(2-Chlor-thiazol-5-yl-methyl)-2-imino-3-nitro-imidazolidin vom Schmelzpunkt 120 °C erhalten.

(Verfahren b)

(b) Analog Beispiel 2 wurden aus 13,1 g (0,05 Mol) 1-(2-Chlor-thiazol-5-yl-methyl)-2-nitroiminoimidazolidin und 50 ml 96 %iger Schwefelsäure 5,2 g (40 % der Theorie) 1-(2-Chlor-thiazol-5-yl-methyl)-2-imino-3-nitro-imidazolidin vom Schmelzpunkt 120 °C erhalten.

Beispiel 4

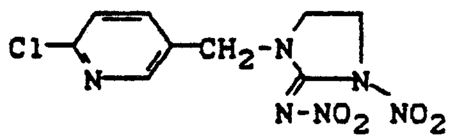

(Verfahren a)

12,8 g (0,05 Mol) 1-(2-Chlor-pyridin-5-yl-methyl)-2-nitroimino-imidazolidin werden bei 15 °C bis 20 °C in 40 ml Eisessig gelöst. Zu dieser Lösung werden bei 20 °C bis 25 °C zunächst 4,8 ml 98 %ige Salpetersäure und dann 14 ml Acetanhydrid tropfenweise gegeben. Das Reaktionsgemisch wird 150 Minuten bei 20 °C gerührt und dann in Eiswasser gegossen. Das kristallin angefallene Produkt wird durch Absaugen isoliert.

Man erhält 13,1 g (87 % der Theorie) 1-(2-Chlor-pyridin-5-yl-methyl)-2-nitroimino-3-nitro-imidazolidin vom Schmelzpunkt 158 °C.

In Analogie zum Beispiel 1 können die folgenden Verbindungen der allgemeinen Formel

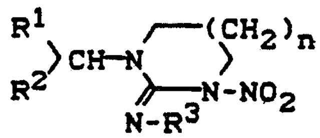 (I)

hergestellt werden:

| Bsp. Nr. | n | $R^1$ | $R^2$ | $R^3$ | Physik. Daten |
|---|---|---|---|---|---|
| 5 | 0 | 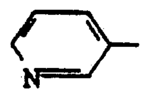 | H | H | |
| 6 | 1 | 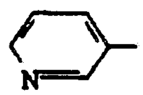 | H | H | |
| 7 | 1 | 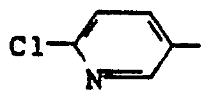 | H | H | |
| 8 | 1 | 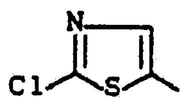 | H | H | |
| 9 | 0 | 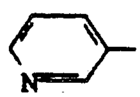 | H | $NO_2$ | |
| 10 | 0 | 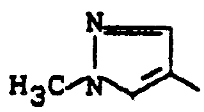 | H | H | |
| 11 | 0 | 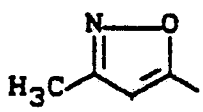 | H | $NO_2$ | |

| Bsp. Nr. | n | $R^1$ | $R^2$ | $R^3$ | Physik. Daten |
|---|---|---|---|---|---|
| 12 | 0 | 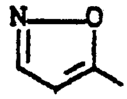 | H | H | |
| 13 | 0 | 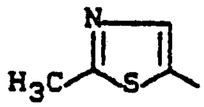 | H | H | |
| 14 | 0 | 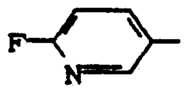 | H | H | |
| 15 | 1 | 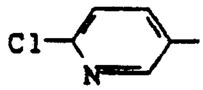 | H | $NO_2$ | |
| 16 | 0 | 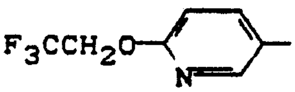 | H | H | |
| 17 | 0 | 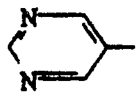 | H | H | |
| 18 | 0 | 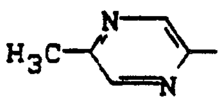 | H | H | |
| 19 | 0 | 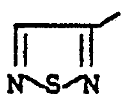 | H | H | |
| 20 | 0 | 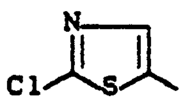 | H | $NO_2$ | |
| 21 | 0 | 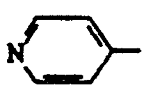 | H | $NO_2$ | |

| Bsp. Nr. | n | $R^1$ | $R^2$ | $R^3$ | Physik. Daten |
|---|---|---|---|---|---|
| 22 | 1 | 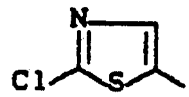 | H | $NO_2$ | |
| 23 | 0 | 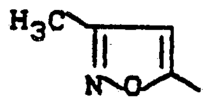 | H | H | |
| 24 | 1 | 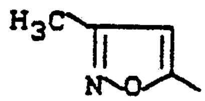 | H | $NO_2$ | |
| 25 | 0 | 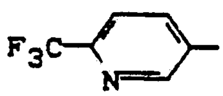 | H | H | |
| 26 | 0 | 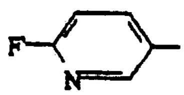 | H | $NO_2$ | |
| 27 | 0 | 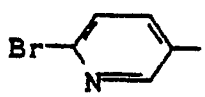 | H | H | |

Verwendungsbeispiele

Beispiel A

Myzus-Test

| | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem test zeigte z.B. die gemäß den Herstellungsbeispielen 1 und 2 erhaltene Verbindung überlegene Wirksamkeit gegenüber dem Stand der Technik.

## Ansprüche

1. 3-Substituierte 1-Nitro-2-imino-1,3-diazacycloalkane der allgemeinen Formel (I)

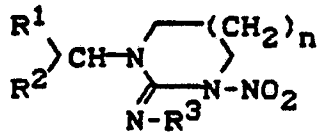

(I)

in welcher
n für die Zahlen 0 oder 1 steht,
$R^1$ für eine fünf- oder sechsgliedrige heterocyclische Gruppierung steht, welche 1, 2, 3 oder 4 Stickstoffatome und/oder ein oder zwei Sauerstoff- oder Schwefelatome als Heteroatom-Ringglieder enthält - wobei die Zahl der Heteroatome 1, 2, 3 oder 4 beträgt -und welche gegebenenfalls durch Halogen, Cyano, Nitro, Alkyl, Halogenalkyl, Alkenyl, Halogenalkenyl, Alkinyl, Alkoxy, Halogenalkoxy, Alkenyloxy, Halogenalkenyloxy, Alkinyloxy, Alkylthio, Halogenalkythio, Alkenylthio, Halogenalkenylthio, Alkinylthio, Alkylsulfinyl, Halogenalkylsulfinyl, Alkylsulfonyl, Halogenalkylsulfonyl, Amino, Alkylamino, Dialkylamino, Aryl, Aryloxy, Arylthio, Arylamino, Aralkyl, Formylamino, Alkylcarbonylamino, Formyl, Carbamoyl, Alkylcarbonyl und/oder Alkoxycarbonyl substituiert ist,
$R^2$ für Wasserstoff oder Alkyl steht und
$R^3$ für Wasserstoff oder Nitro steht.

2. 3-Substituierte 1-Nitro-2-imino-1,3-diazacycloalkane der allgemeinen Formel (I) gemäß Anspruch 1, in welcher
n für die Zahlen 0 oder 1 steht,
$R^1$ für eine fünf- bzw. sechsgliedrige heterocyclische Gruppierung aus der Reihe Furyl, Thienyl, Pyrrolyl, Pyrazolyl, Imidazolyl, 1,2,3- oder 1,2,4-Triazolyl, Oxazolyl, Isoxazolyl, 1,2,4- oder 1,3,4-Oxadiazolyl, Thiazolyl, Isothiazolyl, 1,2,3-, 1,2,4,-1,2,5-oder 1,3,4-Thiadiazolyl, Pyridyl, Pyridazinyl, Pyrimidinyl und Pyrazinyl steht, welche gegebenenfalls durch Fluor, Chlor, Brom, Iod, Cyano, Nitro, $C_1$-$C_4$-Alkyl (welches gegebenenfalls durch Fluor und/oder Chlor substituiert ist), $C_2$-$C_4$-Alkenyl (welches gegebenenfalls durch Fluor und/oder Chlor substituiert ist), $C_2$-$C_4$-Alkinyl, $C_1$-$C_4$-Alkoxy (welches gegebenenfalls durch Fluor und/oder Chlor substituiert ist), $C_3$-$C_4$-Alkenyloxy (welches gegebenenfalls durch Fluor und/oder Chlor substituiert ist), $C_3$-$C_4$-Alkinyloxy, $C_1$-$C_4$-Alkylthio (welches gegebenenfalls durch Fluor und/oder Chlor substituiert ist), $C_3$-$C_4$-Alkenylthio (welches gegebenenfalls durch Fluor und/oder Chlor substituiert ist), $C_3$-$C_4$-Alkinylthio, $C_1$-$C_4$-Alkylsulfinyl (welches gegebenenfalls durch Fluor und/oder Chlor substituiert ist), $C_1$-$C_4$-Alkylsulfonyl (welches gegebenenfalls durch Fluor und/oder Chlor substituiert ist), Amino, $C_1$-$C_4$-Alkylamino, Di-($C_1$-$C_4$-alkyl)-amino, Phenyl, Phenoxy, Phenylthio, Phenylamino, Benzyl, Formylamino, $C_1$-$C_4$-Alkylcarbonylamino, Formyl, Carbamoyl, $C_1$-$C_4$-Alkylcarbonyl und/oder $C_1$-$C_4$-Alkoxy-carbonyl substituiert ist,
$R^2$ für Wasserstoff oder $C_1$-$C_3$-Alkyl steht und
$R^3$ für Wasserstoff oder Nitro steht.

3. 3-Substituierte 1-Nitro-2-imino-1,3-diazacycloalkane der allgemeinen Formel (I) gemäß Anspruch 1, in welcher
n für die Zahlen 0 oder 1 steht,
$R^1$ für eine fünf- bzw. sechsgliedrige heterocyclische Gruppierung aus der Reihe Pyrazolyl, 1,2,4-Triazolyl, Oxazolyl, Isoxazolyl, Thiazolyl, Isothiazolyl, 1,2,5-Thiadiazolyl, Pyridyl, Pyrazinyl und Pyrimidinyl steht, welche gegebenenfalls durch Fluor, Chlor, Brom, Cyano, Nitro, $C_1$-$C_2$-Alkyl (welches gegebenenfalls durch Fluor und/oder Chlor substituiert ist), $C_1$-$C_2$-Alkoxy (welches gegebenenfalls durch Fluor und/oder Chlor substituiert ist), $C_1$-$C_2$-Alkylthio (welches gegebenenfalls durch Fluor und/oder Chlor substituiert ist) oder $C_1$-$C_2$-Alkylsulfonyl (welches gegebenenfalls durch Fluor und/ oder Chlor substituiert ist) substituiert ist,
$R^2$ für Wasserstoff steht,
$R^3$ für Wasserstoff oder Nitro steht.

4. Verfahren zur Herstellung von 3-substituierten 1-Nitro-2-imino-1,3-diazacycloalkanen der allgemeinen Formel (I)

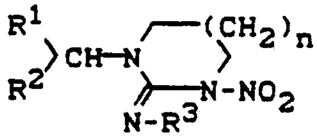

(I)

in welcher
n für die Zahlen 0 oder 1 steht,
$R^1$ für eine fünf- oder sechsgliedrige heterocyclische Gruppierung steht, welche 1, 2, 3 oder 4- Stickstoffatome und/oder ein oder zwei Sauerstoff- oder Schwefelatome als Heteroatom-Ringglieder enthält - wobei die Zahl der Heteroatome 1, 2, 3 oder 4 beträgt -und welche gegebenenfalls durch Halogen, Cyano, Nitro, Alkyl, Halogenalkyl, Alkenyl, Halogenalkenyl, Alkinyl, Alkoxy, Halogenalkoxy, Alkenyloxy, Halogenalkenyloxy, Alkinyloxy, Alkylthio, Halogenalkythio, Alkenylthio, Halogenalkenylthio, Alkinylthio, Alkylsulfinyl, Halogenalkylsulfinyl, Alkylsulfonyl, Halogenalkylsulfonyl, Amino, Alkylamino, Dialkylamino, Aryl, Aryloxy, Arylthio, Arylamino, Aralkyl, Formylamino, Alkylcarbonylamino, Formyl, Carbamoyl, Alkylcarbonyl und/oder Alkoxycarbonyl substituiert ist,
$R^2$ für Wasserstoff oder Alkyl steht und
$R^3$ für Wasserstoff oder Nitro steht,
dadurch gekennzeichnet, daß man
(a) 1-substituierte 2-Imino-1,3-diaza-cycloalkane der allgemeinen Formel (II),

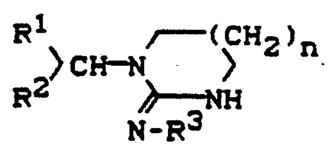

(II)

in welcher
n, $R^1$, $R^2$ und $R^3$ die oben angegebenen Bedeutungen haben,
-oder deren Hydrochloride-
mit einem Nitrierungsmittel, gegebenenfalls in Gegenwart eines Katalysators und gegebenenfalls in Gegenwart eines Verdünnungsmittels umsetzt, oder daß man
-für den Fall, daß $R^3$ für Wasserstoff steht-
(b) 1-substituierte 2-Nitroimino-1,3-diazacycloalkane der allgemeinen Formel (IIa),

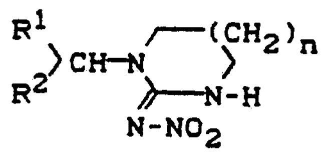

(IIa)

in welcher
n, $R^1$ und $R^2$ die oben angegebenen Bedeutungen haben,
gegebenenfalls in Gegenwart eines Katalysators und gegebenenfalls in Gegenwart eines Verdünnungsmittels, isomerisiert.

5. Schädlingsbekämpfungsmittel, gekennzeichnet durch einen Gehalt an mindestens einem 3-substituierten 1-Nitro-2-imino-1,3-diazacycloalkan der Formel (I).

6. Insektizide Mittel, gekennzeichnet durch einen Gehalt an mindestens einem 3-substituierten 1-Nitro-2-imino-1,3-diazacycloalkan der Formel (I).

7. Verfahren zur Bekämpfung von Insekten, dadurch gekennzeichnet, daß man 3-substituierte 1-Nitro-2-imino-1,3-diazacycloalkane der Formel (I) auf Insekten und/oder deren Lebensraum einwirken läßt.

8. Verwendung von 3-substituierten 1-Nitro-2-imino-1,3-diazacycloalkanen der Formel (I) zur Bekämpfung von Insekten.

9. Verfahren zur Herstellung von Schädlingsbekämpfungsmitteln, dadurch gekennzeichnet, daß man 3-substituierte 1-Nitro-2-imino-1,3-diazacycloalkane der Formel (I) mit Streckmitteln und/oder oberflächenaktiven Mitteln vermischt.

Europäisches Patentamt

# EUROPÄISCHER RECHERCHENBERICHT

Nummer der Anmeldung

EP 89108651.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | US - A - 4 297 496 (DAVIS) * Zusammenfassung * | 1,5-9 | C 07 D 401/06<br>C 07 D 403/06<br>C 07 D 405/06<br>C 07 D 409/06<br>C 07 D 413/06<br>C 07 D 417/06<br>A 01 N 43/54 |
| A | EP - A1 - 0 192 060 (NIHON) * Ansprüche 1,4; Tabelle 7-11 * | 1 | |
| A | CHEMICAL ABSTRACTS, Band 51, Nr. 3, 10. Februar 1989, Columbus, Ohio, USA CARL BOYARS et al. "Kinetics and mechanism of the cycliza--tion of substituted alkylnitroguanidines" Spalte 1843, Zusammenfassung-Nr. 1 843i & J. Am. Chem. Soc. 78, 4590-5(1956) | 1 | |
| A | CHEMICAL ABSTRACTS, Band 54, Nr. 21, 10. November 1960, Columbus, Ohio, USA W.A. SKINNER et al. "Potential anticancer agents. XXXI. Relationship of chemical structure to antileukemic activity with analogs of 1-methyl-3-nitro-1 nitrosogua--nidine (NSC-9369)" Spalte 22595, Zusammenfassung-Nr. 22 595a & J. Med. Pharm. Chem. 2, 299-333 (1960) | 1 | RECHERCHIERTE SACHGEBIETE (Int. Cl.4)<br>C 07 D 401/00<br>C 07 D 403/00<br>C 07 D 405/00<br>C 07 D 409/00<br>C 07 D 413/00<br>C 07 D 417/00 |
| P,A | CHEMICAL ABSTRACTS, Band 110, Nr. 1, 2. Jänner 1989, Columbus, Ohio, USA TSUBOI, SHINICHI et al. | 1,5-9 | |

Der vorliegende Recherchenbericht wurde für alle Patentansprüche erstellt.

| Recherchenort | Abschlußdatum der Recherche | Prüfer |
|---|---|---|
| WIEN | 04-08-1989 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

# EUROPÄISCHER RECHERCHENBERICHT

Nummer der Anmeldung



EP 89108651.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| | "Agricultural synergistic insecticides containing heterocycles" Seite 272, Spalte 1, Zusammenfassung-Nr. 2 918u & Jpn. Kokai Tokkyo Koho JP 63,126,804 [88,126,804] 1988 -- | | |
| P,A | CHEMICAL ABSTRACTS, Band 109, Nr. 25, 19. Dezember 1988, Columbus, Ohio, USA TSUBOI, SHINICHI et al. "Agricultural synergistic insecticides containing heterocycles and carbamates" Seite 295, Spalte 1, Zusammenfassung-Nr. 224 736b & Jpn. Kokai Tokkyo Koho JP 63,126,806 [88,126,806] 30 May 88 -- | 1,5-9 | |
| P,A | CHEMICAL ABSTRACTS, Band 109, Nr. 25, 19. Dezember 1988, Columbus, Ohio, USA TSUBOI, SHINICHI et al. "Agricultural synergistic insecticides containing heterocycles and phosphates" Seite 295, Spalte 1, Zusammenfassung-Nr. 224 737c & Jpn. Kokai Tokkyo Koho JP 63,126,810 [88,126,810] 30 May 1988 ---- | 1,5-9 | RECHERCHIERTE SACHGEBIETE (Int. Cl.4) |

Der vorliegende Recherchenbericht wurde für alle Patentansprüche erstellt.

| Recherchenort | Abschlußdatum der Recherche | Prüfer |
|---|---|---|
| WIEN | 04-08-1989 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument